# EUROPEAN PATENT APPLICATION

(11) **EP 4 825 187 A1**
(43) Date of publication of application: **21.10.2026**
(21) Application number: 25171054.7
(22) Date of filing: 16.04.2025
(51) Int. Cl.: A23J 7/00, A61K 31/05, C07F 9/10

(54) **PURIFIED EGG-YOLK LECITHIN AND PROCESS OF PREPARATION THEREOF**

(71) Applicant: Fresenius Kabi Deutschland GmbH, 61352 Bad Homburg (DE)
(72) Inventor: SINGH TOMAR, Vinod, 122 101 Gurgaon, Haryana (IN); NITIN, Gupta, 122 101 Gurgaon, Haryana (IN); CABRI, Walter, 20051 Cassina de' Pecchi - MILANO (IT); ABUL, Azim, 122 101 Gurgaon, Haryana (IN); RICCI, Antonio, 20051 Cassina de' Pecchi - MILANO (IT); SINGH, Govind, 122 001 Gurgaon (IN)
(74) Representative: Fresenius Kabi Deutschland GmbH

(57) **Abstract**

The present invention provides a purified egg-yolk lecithin and its process of preparation by using environment friendly solvents and conditions and therefore, reducing the environmental footprint. The present invention further provides a lipid emulsion comprising purified egg-yolk lecithin obtained by the process of present invention.

## Description

### FIELD OF THE INVENTION

The present invention relates to a purified egg-yolk lecithin and a process of preparation thereof. Further, the present invention relates to a lipid emulsion comprising the purified egg-yolk lecithin obtained by the process of the present invention and a propofol composition containing such a lipid emulsion.

### BACKGROUND OF THE INVENTION

Egg-yolk lecithin is a natural phospholipid mixture derived from extraction and refinement of egg-yolk. Historically, it has been used as an emulsifier in the food and pharmaceutical industries. An egg-yolk lecithin, enriched in phosphatidylcholine, serves as an additive in pharmaceuticals, particularly as an emulsifier in fat emulsions.

During the extraction of phospholipids from egg-yolk, impurities such as cholesterol and neutral lipids are also co-extracted. Various methods exist to purify egg-yolk lecithin including solvent extraction, chromatography, and low-temperature solvent precipitation. The choice of purification method depends on the intended use of the isolated lecithin.

One method for the isolation of egg-yolk lecithin from egg-yolk is based on the use of acetone. Because, egg-yolk lecithin is only poorly soluble in acetone, it precipitates, whereas impurities such as a neutral lipids dissolve in acetone and are separated.

EP2502928B1 discloses a method for producing a purified egg-yolk phospholipid by treatment with water and alcohol containing acetone. The acetone content is 80% and the alcohol content is 0.3 to 3 vol%. However, the yield of phosphatidylcholine enriched phospholipid is not high enough for the industrial application in this rather inefficient process.

Luz Palacios and Tong Wang (Journal of American Oil Chemists Society (2005) 82,565-569) disclosed an extraction of de-oiled and undeoiled egg-yolk lecithin with ethanol. The purity of the phosphatidylcholine enriched egg-yolk lecithin achieved is quite low though (35.7-53.3%). The method also uses several organic solvents, including a chlorinated solvent, such as chloroform, making it a non-environmentally friendly process.

Current extraction methods which involve the use of acetone often result in egg-yolk lecithin that contains significant amounts of residual impurities.

Even if some neutral lipids can be reduced sufficiently, to obtain a purified egg-yolk lecithin satisfying the requirements of a component for producing an emulsion of pharmaceutical grade, it may be necessary to repeat the treatment with additional solvents many times, thereby increasing time and cost and also reducing the egg-yolk lecithin yield.

These traditional extraction processes involve the use of organic solvents that can have negative environmental impacts if not properly managed, leading to environmental and safety concerns.

Egg-yolk lecithin, derived from egg yolk is commercially available and may be used in some dedicated pharmaceutical and food applications.

The presence of certain impurities is considered a major contributor to the colour of egg-yolk lecithin.

It was considered important in the industry that already the eggs used as starting material for the process of egg-yolk lecithin isolation or purification are low in certain pigments. The inventors were therefore looking for an efficient purification process that allows the use of a broader variety of eggs, and therefore also a broader variety of egg-yolk powder (such as SFE-EYP), as source or starting material for the extraction and isolation process. Thereby omitting the need for selecting for those eggs only, which comprise a lower amount of colouring pigments.

As egg-yolk lecithin is used as an emulsifier for pharmaceutical or nutritional emulsions, the presence of these impurities also contributes to the appearance of such emulsions. The inventors of this process for preparing purified egg-yolk lecithin from SFE-EYP have found that a decolourization with activated charcoal alone can remove coloured impurities to some extent only, resulting in final egg-yolk lecithin still showing a significant unspecific absorption due to presence of such remaining impurities in the egg-yolk lecithin.

Propofol is an alkylphenol derivative (2,6-di-isopropyl- phenol) marketed as an oil water emulsion using soybean oil (10%), and egg lecithin (1.2%) as the emulsifying agent.

Therefore, there is a need in the art to develop a process for the isolation of egg-yolk lecithin in a purity that is sufficient for the preparation of pharmaceutical grade emulsions, such as an emulsion of propofol, which provides for a sufficient yield of desired product, resulting in white appearance, and which is allowing for a reduction, or avoidance, of the use of environmentally harmful solvents.

### OBJECT OF THE INVENTION

The primary objective of the present invention is to address and overcome one or more disadvantages in the prior art processes for isolating an egg-yolk lecithin resulting in an egg-yolk lecithin with reduced amounts of impurities.

Another objective of the present invention is to provide a purified egg-yolk lecithin.

Yet another objective of the present invention is to provide a process for the preparation of purified egg-yolk lecithin that yields a sufficient amount of purified egg-yolk lecithin to be applicable in the industrial production.

Yet another objective of the present invention is to provide a process for the preparation of purified egg-yolk lecithin using environment friendly solvents and conditions, and thereby reducing the environmental footprint and ensuring the safety of the production process of the purified egg-yolk lecithin.

A further objective of the present invention is to provide a lipid emulsion comprising the purified egg-yolk lecithin.

A further objective of the present invention is to provide a process to prepare a pharmaceutical emulsion which uses the egg-yolk lecithin obtained by the new process as emulsifier to obtain white and stable pharmaceutical emulsions.

### SUMMARY OF THE INVENTION

The present invention relates to a purified egg-yolk lecithin and its process of preparation. The present invention further relates to a lipid emulsion comprising the purified egg-yolk lecithin obtained by the process of present invention. It further relates to a process of preparing such a lipid emulsion.

**In a first aspect,** the present invention provides a process for the preparation of a purified egg-yolk lecithin comprising the steps of:
**a.** providing supercritical fluid extracted egg-yolk powder as starting material,
**b.** treating the supercritical fluid extracted egg-yolk powder with a solvent,
**c.** separating the product obtained in step b) from the solvent used in step b,
**d.** extracting an egg-yolk lecithin fraction from the product obtained in step c),
**e.** optionally, treating the egg-yolk lecithin fraction obtained in step d) with charcoal,
**f.** precipitating impurities from the egg-yolk lecithin fraction obtained in step d) or step e),
**g.** removing the precipitated impurities from the product obtained in step f),
**h.** isolating the purified egg-yolk lecithin from the product obtained in step g), and
**i.** optionally, drying the purified egg-yolk lecithin;
   wherein, in step b) the solvent is dimethyl carbonate.

**In a second aspect,** the present invention provides the purified egg-yolk lecithin obtained by the process as described above.

**In a third aspect,** the present invention provides a nutritional product comprising a purified egg-yolk lecithin obtained by the process of the present invention.

**In a fourth aspect,** the present invention provides a lipid emulsion comprising a purified egg-yolk lecithin obtained by the process of the present invention. It may contain a pharmaceutically active ingredient and may be used as pharmaceutical composition.

**In a fifth aspect,** the present invention provides a method for preparing a lipid emulsion, comprising the steps of:
**a.** providing an oil phase comprising soybean oil, a lipophilic, pharmaceutically active ingredient, preferably, propofol, and optionally a pharmaceutically acceptable antioxidant;
**b.** providing an aqueous phase comprising water for injection and optionally a pharmaceutically acceptable tonicity agent, and an agent for pH adjustment, and optionally a pharmaceutically acceptable co-emulsifier, preferably a free long chain fatty acid, more preferably an oleic acid;
**c.** forming a pre-emulsion by mixing the oil phase provided in step a) with the aqueous phase provided in step b);
**d.** forming an emulsion by high-pressure homogenizing the pre-emulsion obtained in step c); and
**e.** sterilizing the emulsion obtained in step d), wherein optionally the emulsion is filled into a suitable container before or after sterilization;
   wherein the purified egg-yolk lecithin according to present invention is added in step a) or step b).

**In a sixth aspect,** the present invention provides an oil-in-water emulsion for parenteral administration, containing:
10 to 20 mg/ml of propofol;
50 to 100 mg/mL soybean oil;
22.5 mg/ml glycerol;
12 mg/ml of purified egg-yolk lecithin according to the present invention;
oleic acid;
optionally, 50 mg/ml of medium-chain triglyceride;
optionally, 0.05 mg/ml of disodium edetate anhydrous (equivalent to 0.055 mg of disodium edetate); sodium hydroxide to adjust pH, and water for injection.

### BRIEF DESCRIPTION OF THE DRAWING

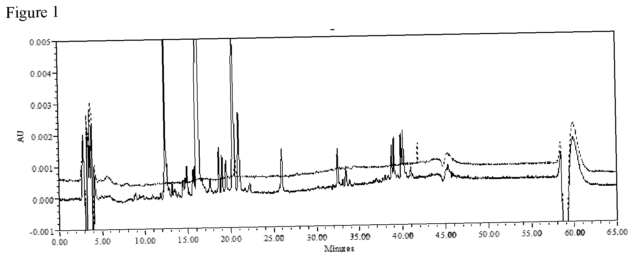

**Fig** 1 is a HPLC chromatogram analysed by HPLC-UV analysis as described in Example 5 showing the UV absorption at 447 nm for purified egg-yolk lecithin that was purified without a treatment with dimethyl carbonate, depicted as solid line, and for purified egg-yolk lecithin that was purified with a treatment with dimethyl carbonate, in step b) of the process according to the present invention, depicted as dotted line.

### DETAILED DESCRIPTION OF THE INVENTION

The present invention provides a process for the preparation of a purified egg-yolk lecithin and its use in the composition. This process has several advantages such as the use of green solvents and the obtainment of highly pure egg-yolk lecithin. Various methods for purification of egg-yolk lecithin are already known in the art. However, the processes known in the art have some disadvantages. Therefore, the present invention is focused on the use of an environmentally friendly solvent, i.e. dimethyl carbonate, for purification of egg-yolk lecithin.

### DEFINITIONS

The following definitions are used in connection with the present application unless the context indicates otherwise.

As used herein, the terms "comprises", "comprising", "includes", "including", "containing", "having" or any other variation thereof, are intended to cover a nonexclusive inclusion.

Dimethyl carbonate, also referred to as DMC, is a carbonate ester that is carbonic acid in which both hydrogens are replaced by methyl groups.

As used herein, the term **"treating"** refers to the process of contacting a substance, such as, for example, supercritical fluid extracted egg-yolk powder, with one or more solvents or other agents. This treatment may involve various methods, including but not limited to, mixing, stirring, agitating, immersing, or exposing to allow for physical interaction between the substance and the solvents or agents. The result of treating can include purifying, extracting, reacting, or otherwise modifying the substance to achieve the desired outcome.

As used herein, the term **"separating"** refers to a process for isolating an insoluble material from a solvent or solution. This process can be accomplished through various techniques, including but not limited to filtration, decantation, extraction, distillation, evaporation, centrifugation, drying, or a combination thereof.

The term **"purified egg-yolk lecithin"** as used herein refers to egg-yolk lecithin that is free from certain impurities and significantly reduced in other impurities. The purified egg-yolk lecithin is further characterized by being enriched in phosphatidylcholine. It presents a purity profile and stability that makes it suitable for further applications in nutritional and pharmaceutical products.

The term **"phosphatidylcholine"** refers to a class of phospholipids that incorporate choline as a headgroup. Hence it refers to variety of compounds, rather than a unique compound, due to several different fatty acid chain types.

The term **"supercritical fluid extracted egg-yolk powder"** or "SFE-EYP" refers to egg-yolk powder where neutral lipids (such as cholesterols) have been extracted using a technique called "supercritical fluid extraction". This technique most commonly uses non-hazardous carbon dioxide as fluid, at conditions above its critical point, where it behaves like both a gas and a liquid, allowing selective extraction based on solubility. Under high pressure and temperature, the extraction results in a powder with potentially reduced fat content while preserving valuable components like phospholipids, depending on the extraction conditions used. Supercritical fluid can selectively extract certain lipids like phospholipids (mainly triglycerides and cholesterol) from the egg-yolk under mild conditions. The supercritical fluid extracted egg-yolk powder is used as starting material in the process according to the invention. It may also be known as defatted or de-oiled egg-yolk powder. A detailed description of the process is disclosed in Hietaniemi (2009) Eur Food Res Technol 228: pages 857-863.

Accordingly, supercritical fluid extracted egg-yolk powder (SFE-EYP) which is referred to herein as starting material for the isolation of egg-yolk lecithin is a composition that is obtained by applying supercritical fluid extraction with CO2 to egg-yolk. Preferably, it is a composition comprising phosphatidylcholine (PC), phosphatidylethanolamine (PE), cholesterol, sphingomyelin (SHP), phosphatidylinositol (PI), lysophosphatidylethanolamine (LPE) and lysophosphatidylcholine (LPC), and various impurities, more preferably it has a reduced content of neutral lipids, more preferably of triglycerides, and cholesterol, as compared to dried egg-yolk powder.

The exact composition of the SFE-EYP depends on the type of egg-yolk used as well as on the conditions used in this extraction process.

Preferably, the SFE-EYP used as starting material for the process of present invention comprises cholesterol below 2% w/w, residual triglyceride 1-2% w/w and more than 70% w/w phosphatidylcholine.

The terms **"extracting" or "extraction"** as used herein refer to a process involving the use of solvents to isolate a specific substance from a mixture of components. This process typically involves dissolving the desired substance in a solvent or solvent mixture, allowing it to separate from the other components in the mixture that are insoluble in such solvent or solvent mixture. The solvent can then be removed to obtain the purified substance. In the process according to the invention the term extracting as used in step d refers to this understanding.

The term **"precipitating"** or **"precipitation"** as used herein refers to a process wherein a substance is solidified. It may crystallize or otherwise change its status from liquid to solid. Precipitation can be induced by changes in temperature, pH, concentration, or the addition of another reagent or solvent.

As used herein, the term "drying" refers to the process of removing residual amounts of liquid, such as a solvent, from a substance, typically by using heat, airflow, vacuum, or a combination of these methods.

**"Medium chain triglyceride"** as used herein refers to a triglyceride with two or three fatty acids having an aliphatic tail of 6-12 carbon atoms.

A **UV-Vis spectrophotometer** is an analytical instrument that measures the amount of ultraviolet (UV) and visible light that is absorbed by a sample. UV-Vis spectrophotometers work by passing a beam of light through the sample and measuring the amount of light that is absorbed at each wavelength. The amount of light absorbed is proportional to the concentration of the absorbing compound in the sample. It measures the intensity of light after passing through a sample (*I*), and compares it to the intensity of light before it passes through the sample (*I*ₒ). The ratio *I*/*I*o is called the *transmittance* and is usually expressed as a percentage (%T). The absorbance, A, is based on transmittance: $A = - log \left(%T/100%\right)$

It is a widely used technique in chemistry, biochemistry, and other fields, to identify and quantify compounds in a variety of samples. Sometimes the UV spectrometer is an inbuild part of an HPLC device.

High-performance liquid chromatography (HPLC) is a technique used to separate molecules based on size and surface charge, among other properties. The incorporation of ultra-violet (UV) spectroscopy with HPLC allows to determine the concentration of molecules identified and separated or simply to compare the relative concentration of same eluents in different runs. Throughout the application when it is referred to a detection at a wavelength of 447 nm that is understood to comprise a detection at wavelength in the range of 445 nm to 450 nm.

The incorporation of a charged aerosol detector (CAD) with HPLC allows to determine the mass of chemical compounds present in a sample by creating charged aerosol particles which are detected using an electrometer. The employed process generates a signal directly proportional to the mass of the analyte present. It is commonly used for the analysis of compounds that cannot be detected using traditional UV/Vis approaches due to their lack of a chromophore.

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. In case of conflict, the present specification, including definitions, will control. In addition, the materials, methods, and examples are illustrative only and not intended to be limiting.

When an amount, concentration, or other value or parameter is given as either a range, preferred range, or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

Egg-yolk is composed of various nutrients, including proteins, fats, and phospholipids. According to Aro et al. (Eur Food Res Technol (2009) 228:857-863) approximately 50% of yolk is water and after spray drying, the resulting powder contains about 60% of lipids, which consist of neutral lipids (65%), phospholipids (31%) and cholesterol (4%). Whereas dried egg-yolk powder has also been described to contain approximately 15% phospholipids, which are mainly composed of roughly 70% phosphatidylcholine and 20% phosphatidylethanolamine (Burley R.W., Vadehra D.V., The Avian Egg Chemistry and Biology, John Wiley & Sons (1989)). It seems undisputed though, that the primary phospholipids in egg-yolk are phosphatidylcholine (PC), constituting about 70% and phosphatidylethanolamine (PE) constituting around 15% of the total phospholipids in egg-yolk. Egg-yolk proteins include lipoproteins and phosphoproteins, which together can make up about 16% of the yolk's weight. However, as reported in the literature, there is considerable variation in the composition of egg yolks, possibly because of differences in the breeds and feed of the hens.

Even if SFE-EYP majorly comprises organic solvent soluble phospholipids and organic solvent soluble proteins and water insoluble proteins, it may also contain various impurities such as natural pigments like carotene and xanthophylls (lutein, canthaxanthin and zeaxanthin) and the like. These impurities may be responsible for the dark yellow to orange colour of egg-yolk lecithin which does impact the usability of the composition for those pharmaceutical and nutritional products which rely on a clear white appearance. These impurities thereby impact the quality and appearance of lipid emulsion composition. It was found that the use of a washing step with a solvent, such as dimethyl carbonate, applied before extraction of the egg-yolk lecithin from the SFE-EYP helps removing these impurities.

The inventors of the present application tried different strategies to control or remove such impurities during a process of isolating purified egg-yolk lecithin from SFE-EYP. They treated the SFE-EYP with several different organic solvents and also tested these in combination with activated charcoal treatment.

While searching for a more environmentally friendly solvent (green solvent) that offers benefits for workplace safety, health, and the environment (e.g., less hazardous waste), various factors needed to be considered, such as the solubility profile of egg-yolk lecithin in the solvent, a low boiling temperature of the solvent, non-reactivity towards phospholipids, and good recoverability. Based on these characteristics, dimethyl carbonate, acetone, methyl acetate and di-isopropyl ether were considered most suitable candidates for purification of SFE-EYP.

Surprisingly, when testing these different solvents, the inventors of the present application found that treating the supercritical fluid extracted egg-yolk powder with dimethyl carbonate not only worked, i.e. purified the SFE-EYP, thereby providing a more environmentally friendly alternative to existing processes but also improved the impurity profile of egg-yolk lecithin without significant reduction of yield, and without creating additional unwanted side products. Creation of such side products could be observed when acetone was used to treat the SFE-EYP, thereby generating new impurities, likely caused by a reaction of the amine group in phosphatidylethanolamine (PE) with acetone.

The impact of using different solvents for the purification of SFE-EYP on the purity of the final egg-yolk lecithin (i.e. purified egg-yolk lecithin after all steps of the process described above have been performed), is shown in Table 1. The table presents the yield (in w/w) achieved, the amount of phosphatidylcholine (PC), and phosphatidylethanolamine (PE) in %w/w by HPLC-CAD, and of colour causing impurities in terms of UV-visible spectrophotometer absorbance at 447 nm:

**Table 1:**

| **Sample No.** | **Solvent** | **Egg-yolk lecithin** | | | **Process yield** |
|---|---|---|---|---|---|
| | | **UV absorption (447 nm)** | **PC (%w/w)** | **PE (%w/w)** | **Yield (w/w)** |
| 1 | None | 0.33 | 78.35 | 6.16 | 0.26 |
| **2** | **Dimethyl carbonate** | **0.009** | **79.38** | **5.60** | **0.27** |
| 3 | Acetone | 0.011 | 77.09 | 6.17 | 0.26 |
| 4 | Methyl acetate | 0.007 | 78.94 | 6.15 | 0.22 |
| 5 | Di-isopropyl ether | 0.007 | 81.15 | 5.27 | 0.20 |

The samples analysed in this UV absorption assay were of the same concentration of purified egg yolk lecithin (final).

The inventors of the present application found that the use of the environmentally friendly solvent dimethyl carbonate in step b) of the process according to the invention is highly effective for reducing impurities from the supercritical fluid extracted egg-yolk powder, preferably those impurities which are considered to cause the yellow to brown colouring, which is considered detrimental to the suitability of egg-yolk lecithin for preparing white pharmaceutical emulsions, such as the propofol emulsions.

The process comprising the treatment with dimethyl carbonate (DMC) provided a very good yield, and an excellent purity profile. UV absorption values at 447 nm are significantly reduced. The process also achieves a product that is enriched in phosphatidylcholine (PC) and reduced in phosphatidylethanolamine (PE) in the purified egg-yolk lecithin.

The UV absorption value determined at 447 nm corresponds to the amounts of carotenoids, mainly lutein and zeaxanthin. Accordingly, the treatment of SFE-EYP with dimethyl carbonate reduces carotenoids, mainly lutein and zeaxanthin in the egg-yolk lecithin.

Liquid chromatography analysis (the result thereof is provided in Figure 1), confirms this effect. It also shows the efficient removal of further impurities when using dimethyl carbonate for the purification of SFE-EYP.

**In a first aspect,** the present invention relates to a process for the preparation of a purified egg-yolk lecithin comprising the steps of:
a. providing supercritical fluid extracted egg-yolk powder as starting material,
b. treating the supercritical fluid extracted egg-yolk powder with a solvent,
c. separating the product obtained in step b) from the solvent used in step b),
d. extracting an egg-yolk lecithin fraction from the product obtained in step c),
e. optionally, treating the egg-yolk lecithin fraction obtained in step d) with charcoal,
f. precipitating impurities from the egg-yolk lecithin fraction obtained in step d) or step e),
g. removing the precipitated impurities from the product obtained in step f),
h. isolating the purified egg-yolk lecithin from the product obtained in step g), and
i. optionally, drying the purified egg-yolk lecithin,
   wherein, in step b) the solvent is dimethyl carbonate.

Accordingly, the present invention provides a multi-step process for the preparation of a purified egg-yolk lecithin using SFE-EYP as starting material, and dimethyl carbonate as a solvent, wherein in a first step the SFE-EYP is provided and in a second step the SFE-EYP is treated with dimethyl carbonate (DMC). Treating may involve one or more of mixing, stirring, agitating, immersing, or exposing SFE-EYP powder with dimethyl carbonate. Preferably, the treating comprises adding SFE-EYP to a solution of dimethyl carbonate and stirring the obtained mixture at an elevated temperature. Preferably the temperature is in a range from 30 to 60°C, preferably from 35 to 55°C, most preferably from 40 to 45°C. It is further preferred that the stirring is continued for 50 minutes to 70 minutes, preferably for 50 minutes to 65 minutes, most preferably for 55 to 65 minutes.

In a preferred embodiment, such treating of SFE-EYP powder with dimethyl carbonate results in obtaining a slurry.

The process may be made even more efficient if a radical scavenger is added, particularly when oxidation protection is needed. Preferably, step b), step d) step e) and/or step g) further comprise an addition of a radical scavenger. It is preferred that said radical scavenger is DL-α-Tocopherol. It may be added during step b), during step d), during and after the charcoal treatment in step e), and/or during step g).

The amount of dimethyl carbonate provided to add the SFE-EYP to in step b) may be 2 to 10 mL per g of SFE-EYP, preferably 3 to 8 mL, more preferably 4 to 8 mL per g of SFE-EYP.

Step b) may further comprise the addition of a radical scavenger. Preferably, the radical scavenger is DL-α-Tocopherol.

In step c), the product obtained in step b) is separated from the solvent.

In a preferred embodiment, the desired product obtained in step b) is solid.

The solid product can be separated using a conventional method such as filtration, decantation, centrifugation, or a combination thereof.

In some embodiments, the separation of the product is done by filtration to remove soluble impurities that are solubilized by the solvent.

The resulting product may be washed with dimethyl carbonate.

To remove remaining dimethyl carbonate the product of step c), with or without washing, before adding the extracting solvent in step d) may be further dried. The drying may be carried out under vacuum at 200 to 300 mbar pressure at a temperature in a range from 15 to 35°C preferably, from 20 to 30°C for 4-6 hours.

In step d), an extraction of egg-yolk lecithin fraction from the product obtained in step c) is performed using a solvent.

The extracting solvent in step d) can be selected from the group consisting of C₁-C₃ alcohols, toluene, C₅-C₇ aliphatic alkanes, mixtures of alcohol with nitriles, mixtures of alcohol with dimethyl carbonate, and mixtures of alcohol with diethyl carbonate.

Preferably, the solvent in step d) is selected from the group consisting of methanol, ethanol, propanol, toluene, n-heptane, a mixture of ethanol and acetonitrile, a mixture of ethanol and dimethyl carbonate, and a mixture of ethanol and diethyl carbonate.

The extraction of egg-yolk lecithin fraction with the solvent may be carried out at a temperature in a range from 15 to 45°C preferably from 20°C to 30°C.

In a preferred embodiment, step d) may further comprise the addition of a radical scavenger. The radical scavenger is preferably DL-α-Tocopherol.

In another preferred embodiment, a radical scavenger may be dissolved in the solvent of step d) as described above before the extraction process. The mixture may be heated at a temperature in a range of 50 to 65°C preferably, 55 to 60°C followed by stirring for 30 to 40 minutes.

The mixture may be filtered to remove insoluble particles. The insoluble material may be optionally rinsed with the solvent mixture. The rinsed solution is then added to the filtrate.

Step e) is optional. It involves treating the egg-yolk lecithin fraction obtained in step d) with charcoal, wherein treating comprises stirring the liquid solvent containing the extracted egg-yolk lecithin fraction with added charcoal. The amount of added charcoal in the process may vary from 1% w/w to 5% w/w, preferably 2% to 4% w/w. It is a preferred embodiment wherein the charcoal treatment is performed at a temperature in a range from 30 to 50°C for a time interval of 30 minutes to 3 hours, more preferably at a temperature of 40 to 45°C for a time interval of 1 hour to 2 hours.

Different types of charcoal, strongly acidic or basic, may be used during step e) of the process.

Step e) may comprise the addition of a radical scavenger after separation of the charcoal from the solution. Preferably, the radical scavenger is DL-α-Tocopherol.

The process further comprises step f) which is a step of precipitating impurities, such as PE, from the egg-yolk lecithin fraction obtained in the previous step. It is a preferred embodiment wherein the precipitation is achieved by cooling the egg-yolk lecithin fraction obtained in step d) or step e). It is preferred that the cooling is done at a temperature in a range of -12 to -8 °C preferably -11 to -10°C followed by stirring for 60 to 90 min, preferably for 90 min, at the reduced temperature to precipitate further impurities.

Step g) comprises the separation of the precipitated impurities from the egg-yolk lecithin containing solution. If the optional step e) is performed prior to the step f), then the removal of the charcoal adsorbed with further impurities may be performed simultaneously. The precipitated impurities may be separated by removing them from the egg-yolk lecithin fraction obtained in step d) or step e) by conventional methods such as filtration, centrifugation etc., preferably the precipitated impurities are removed by filtration.

Step g) may involve the addition of a radical scavenger after removal of precipitated impurities from the solution. Preferably, the radical scavenger is DL-α-Tocopherol.

After the removal of the precipitate (cooling and filtration), the product obtained in step g) is a solution.

In step h) of the process the dissolved purified egg-yolk lecithin is isolated from the product obtained in step g). This step may be performed by conventional methods such as concentration, distillation, evaporation etc., preferably purified egg-yolk lecithin is isolated by evaporation. In this step a viscous product is obtained.

The isolated purified egg-yolk lecithin may optionally be dried. With an optional step i) of drying, e.g. by using a belt freeze dryer, the product may be converted into a fluffy solid. Preferably the drying takes place at a temperature in a range of 25°C to 35°C for 1 to 4 hours.

**In a second aspect,** the present invention provides a purified egg-yolk lecithin obtained by the process described above. This purified egg-yolk lecithin is clearly whiter than egg-yolk lecithin prepared by a process not involving a step b) of treating the SFE-EYP with dimethyl carbonate. The purified egg-yolk lecithin has a significantly reduced UV-absorbance value at a retention time where lutein and zeaxanthin are eluting, detected at a wavelength of 447 nm, indicating a significantly reduced amount of zeaxanthin and/or lutein compared to egg-yolk lecithin not prepared by a process involving a step b) of treating the SFE-EYP with dimethyl carbonate. Said UV absorbance value determined as AUC, detected with a UV Vis spectrophotometer at 447 nm, is lower than said UV absorbance of egg-yolk lecithin prepared by a process that only differs by not involving step b). Preferably, said UV absorbance value detected at 447 nm at a retention time where lutein and zeaxanthin are eluting is by a factor 10 lower, more preferably by a factor 25, most preferably by factor 50 lower than said UV absorbance of egg-yolk lecithin prepared by a process that only differs by not involving step b). In a most preferred embodiment said UV absorbance value is by a factor 68 lower than said UV absorbance of egg-yolk lecithin prepared by a process that only differs by not involving step b).

Preferably this purified egg-yolk lecithin obtained by the process according to the invention is free from impurities A, B and C, eluting at a RT of 18.66 min, RT of 19.0 min and a RT of 19.5 min in an HPLC-UV analysis, described in Example 5, wherein the relative retention time of lutein is 16.2. This is shown in table 2.

The data showing the impact of treating the SFE-EYP with dimethyl carbonate or with no solvent, on the final egg-yolk lecithin, in terms of impurities eluting at RT 16.2 min, 18.66 min, 19.0 min and 19.5 min in a HPLC-UV-analysis (described in Example 5), are presented in the following table.

**Table 2:**

| **Product analysed** | **Lutein & Zeaxanthin RT 16.2** | **Impurity A RT 18.66** | **Impurity B RT 19.0** | **Impurity C RT 19.5** |
|---|---|---|---|---|
| Commercially available Egg- yolk lecithin | 23175 | 727 | 747 | 1197 |
| Egg-yolk lecithin (wherein SFE-EYP is purified using DMC) | 827 | 0 | 0 | 0 |
| Egg-yolk lecithin (wherein process involves only Charcoal treatment) | 56399 | 3672 | 6464 | 527 |

The purified egg-yolk lecithin obtained by the process according to the invention also has a better lipid profile in terms of an enrichment in phosphatidylcholine (PC) and a reduction of phophatidylethanolamine (PE), cholesterol, sphingomyelin (SHP), phosphatidylinositol (PI), lysophosphatidylethanolamine (LPE), lysophosphatidylcholine (LPC), as compared to the commercially available product tested.

In yet another preferred embodiment, the purified egg-yolk lecithin obtained by the process, which results in a better yield than when different solvents are used, has an improved purity profile as compared to the product obtained by using different solvents, or compared to alternative processes.

The improved purity profile can be described as reduced amounts of phosphatidylethanolamine, cholesterol, sphingomyelin, phosphatidylinositol, lysophosphatidylethanolamine, and lysophosphatidylcholine.

Data showing the impact of treating SFE-EYP with different solvents in step b) of the process, on the final egg-yolk lecithin, in terms of lipid profile and yield, are presented in **Table 3** and compared with commercially available egg-yolk lecithin. The first row lists the different compounds which were determined. The second to fourth rows present the masses of these compounds present in a commercially available egg-yolk lecithin (batch 1); the masses of these compounds present in egg-yolk lecithin wherein SFE-EYP is purified using acetone in step b of the process (batch 2), and the masses of these compounds present in egg-yolk lecithin wherein SFE-EYP is purified using DMC in step b of the process (batch 3).

**Table 3:**

| **Batch** | **PC (%w/w)** | **PE (%w/w)** | **Choleste rol (%w/w)** | **SPH (%w/w)** | **PI (%w/w)** | **LPE (%w/w)** | **LPC (%w/w)** | **Yield (w/w)** |
|---|---|---|---|---|---|---|---|---|
| (1) | 76.62 | 7.38 | 0.43 | 2.00 | 0 | 0.22 | 1.53 | - |
| (2) | 77.09 | 6.17 | 0.32 | 1.51 | 0 | 0.09 | 1.51 | 0.26 |
| (3) | **79.38** | **5.60** | **0.39** | **1.38** | **0** | **0.09** | **1.48** | **0.27** |

When using DMC as solvent in step b) of the process (3) according to the first aspect of the invention, the PE content in the product obtained is reduced significantly to below 6 %w/w, preferably to 5.6 %w/w and the PC content is enriched significantly to above 78 %w/w, preferably to 79.38 %w/w compared to when acetone is used as solvent in step b) of the process (2), or when compared to a commercially available egg-yolk lecithin (1).

The purified egg-yolk lecithin according to the second aspect of the invention may also comprise traces of dimethyl carbonate. In pilot scale batches the value is as low as between 50 and 110 ppm. This was detected by GC-MS. This is obviously well below the pharmaceutical requirements.

It is a further advantage of the purified egg-yolk lecithin according to the second aspect of the invention that residual amounts of ketone-like organic solvents or chlorinated solvents are present at a level below 10 ppm. Preferably, said ketone-like organic solvents are selected from the group consisting of acetone, butanone (also known as methyl ethyl ketone (MEK)) and methyl isobutyl ketone (MIBK). Preferably the chlorinated solvents are selected from the group consisting of chloroform, dichloromethane and tetrachloromethane. Preferably said purified egg-yolk lecithin is free from any such solvents.

**In a third aspect,** the present invention provides a lipid emulsion comprising the purified egg-yolk lecithin obtained by the process according to the invention. Lipid emulsions may contain egg-yolk lecithin as emulsifying agent. Such a lipid emulsion may be used to provide pharmaceutically active ingredients that are considered essentially insoluble in water for an intravenous application.

These lipid emulsions benefit from the use of the egg-yolk lecithin according to the invention, because this egg-yolk lecithin is free from the impurities described above. Accordingly, it appears whiter and so does the lipid emulsion. Pharmaceutical products that appear yellowish or even have a slight orange tone are not as well accepted by the patient, as are pharmaceutical products that are bright white. Patients tend to associate a lower quality with a lesser shade of white, and compliance might be affected, and even more important, the significant healing effect that is based on the personal trust in the power of the medicine taken, may be reduced, if a patient even subconsciously mistrusts his medication.

Propofol is a highly lipophilic compound and is essentially insoluble in water or other aqueous medium. Therefore, it is formulated as an intravenous emulsion with 10% lipid (1 mL contains 1.1 kcal; 0.1 g fat) containing soybean oil and egg lecithin so that the lipid component is used as a carrier for the drug.

**In a fourth aspect,** the present invention provides a method for preparing a lipid emulsion containing a lipophilic, pharmaceutically active ingredient, preferably propofol, comprising the steps of:
a) providing an oil phase comprising soybean oil, a lipophilic pharmaceutically active ingredient, preferably propofol, and optionally a pharmaceutically acceptable antioxidant,
b) providing an aqueous phase comprising water for injection and optionally a pharmaceutically acceptable tonicity agent, and optionally a pharmaceutically acceptable co-emulsifier, preferably the co-emulsifier being a free long chain fatty acid, more preferably being oleic acid, and an agent for pH adjustment and optionally a chelating agent;
c) forming a pre-emulsion by mixing the oil phase provided in step a) with the aqueous phase provided in step b);
d) forming an emulsion by high-pressure homogenizing the pre-emulsion obtained in step c); and
e) sterilizing the emulsion obtained in step d), wherein optionally the emulsion is filled into a suitable container before or after sterilization,
   wherein the purified egg-yolk lecithin is added in step a) or step b).

The added purified egg-yolk lecithin is the one described above.

The oil phase comprises soybean oil and a lipophilic pharmaceutically active ingredient. Preferably, this pharmaceutically active ingredient is propofol. The oil phase may also comprise a pharmaceutically acceptable antioxidant. Preferably it does not comprise a pharmaceutically acceptable antioxidant.

The oil components, preferably soybean oil and the lipophilic active ingredient propofol may be mixed.

In a preferred embodiment, the oil phase in step a) further comprises medium chain triglycerides.

The aqueous phase may contain a pharmaceutically acceptable tonicity agent, and optionally a pharmaceutically acceptable co-emulsifier and/or an agent for pH adjustment and/or a chelating agent. It is a preferred embodiment wherein the aqueous phase comprises a pharmaceutically acceptable tonicity agent, a pharmaceutically acceptable co-emulsifier and an agent for pH adjustment. It is even more preferred that the pharmaceutically acceptable tonicity agent is glycerol, and the co-emulsifier is a free long chain fatty acid, more preferably an oleic acid.

Oleic acid is a fatty acid, also known as cis-9-octadecenoic acid. Fatty acids (or their salts) often do not occur as such in biological systems. Instead, fatty acids such as oleic acid occur as their esters, commonly triglycerides. As component in this emulsion, however, it is used as free fatty acid.

The components of the aqueous phase may be dispersed in water to obtain a suspension. The suspension may be obtained by mixing at high intensity. In this process, pH adjustment may be carried out. The pH adjustment may be carried out using sodium hydroxide.

The oil phase may be transferred to the aqueous phase through a membrane filter. The aqueous phase components and the oil phase components may be mixed together to obtain a pre-emulsion.

The emulsion may be obtained by performing a high-pressure homogenization on the pre-emulsion.

The process of homogenization may be carried out by applying pressures of 350 and 400 bar at a temperature in a range of 50 to 80°C. This is preferably done at a temperature in a range of 55 to 70°C. This process may be followed by sterilization. The emulsion may be filled into a suitable container before or after sterilization. The emulsion may further be diluted under stirring with appropriate amount of water followed by filtration to obtain the final emulsion.

A propofol emulsion that was made by the method described above, using purified egg-yolk lecithin according to the invention, as described above is surprisingly stable and appears whiter than emulsions made with egg-yolk lecithin not purified according to the invention.

**In a fifth aspect,** the present invention provides an oil-in-water emulsion composition for parenteral administration, containing 10 to 20 mg/ml of propofol, 50 to 100 mg/mL soybean oil, 22.5 mg/ml glycerol, 12 mg/ml of purified egg-yolk lecithin according to the present invention, optionally 0.05 mg/ml of disodium edetate anhydrous (equivalent to 0.055 mg of disodium edetate), sodium hydroxide to adjust the pH, and water for injection.

In a preferred embodiment, the oil-in-water emulsion composition further comprises medium-chain triglycerides in a range of 50 to 100mg/ml.

In further preferred embodiment, the oil-in-water emulsion composition further comprises oleic acid.

In a preferred embodiment, the present application provides an oil-in-water emulsion for parenteral administration, containing:
10.0 mg/ml of propofol,
100.0 mg/mL soybean oil,
22.5 mg/ml glycerol,
12.0 mg/ml of purified egg-yolk lecithin according to the present invention,
0.055 mg/ml of disodium edetate anhydrous (equivalent to 0.05 mg of disodium edetate) as microbial inhibitor, and
sodium hydroxide to adjust pH, and water for injection.

In another preferred embodiment, the present application provides an oil-in-water emulsion for parenteral administration, containing:
10.0 mg/ml of propofol,
100.0 mg/mL soybean oil,
22.5 mg/ml glycerol,
12.0 mg/ml of purified egg-yolk lecithin according to the present invention,
sodium hydroxide to adjust pH, and water for injection.

In yet another preferred embodiment, the present invention provides an oil-in-water emulsion for parenteral administration, containing:
20.0 mg/ml of propofol,
100.0 mg/mL soybean oil,
22.5 mg/ml glycerol,
12 mg/ml of purified egg-yolk lecithin according to the present invention,
sodium hydroxide to adjust pH, and water for injection.

In another preferred embodiment, the present invention provides an oil-in-water emulsion for parenteral administration, containing:
10.0 mg/ml of propofol,
50.0 mg/mL soybean oil,
50 mg/ml of medium-chain triglycerides,
22.5 mg/ml glycerol,
12.0 mg/ml of purified egg-yolk lecithin according to the present invention,
sodium hydroxide to adjust pH, and water for injection.

In another preferred embodiment, the present invention provides an oil-in-water emulsion for parenteral administration, containing:
20 mg/ml of propofol,
50 mg/mL soybean oil,
50 mg/ml medium-chain triglycerides,
22.5 mg/ml glycerol,
12 mg/ml of purified egg-yolk lecithin according to the present invention,
sodium hydroxide to adjust pH, and water for injection.

Preferably, all of these compositions also contain oleic acid.

### ABBREVIATIONS

- SFE-EYP :: supercritical fluid extracted egg-yolk powder
- PC :: phosphatidylcholine
- PE :: phosphatidylethanolamine
- LPE :: Lysophosphatidylethanolamine
- LPC :: Lysophosphatidylcholine
- SPH :: Sphingomyelin
- PI :: Phosphatidylinositol
- HPLC :: High performance liquid chromatography
- LC :: Liquid chromatography
- UV :: Ultraviolet
- w/w :: weight by weight
- DMC :: dimethyl carbonate
- DL :: dextrorotatory and laevorotatory
- Mbar :: millibar
- CAD :: Charged aerosol detector
- MTBE :: 2-Methoxy-2-methylpropan (Methyl-tert-butylether)

### EXPERIMENTAL

Detailed experimental parameters according to the present invention are provided by the following examples, which are intended to be illustrative and not limiting any possible embodiment of the invention.

### EXAMPLES

### Example 1: Preparation process of purified egg-yolk lecithin with DMC wash and charcoal treatment

3540 ml dimethyl carbonate were charged into a 5L reactor. A solution of DL-α-tocopherol (0.15g) in dimethyl carbonate (60 ml) was prepared and added. 600g of supercritical fluid extracted egg-yolk powder was added and mixed with the 3600 ml solution under stirring. The mixture was heated to 40 to 45°C and stirred for 55 to 65 minutes at this temperature. The hot mixture was filtered, using a Buchner funnel with 10µ Whatman filter paper and 10 µ filter cloth. The filtered solid was washed with 1200 ml (600 mLx2) of dimethyl carbonate. The solid was dried under vacuum 200-300 mbar (610 to 535 mmHg) at 20 to 30°C for 4 to 6 hours.

The resulting DMC washed SFE-EYP was extracted using 3540 mL of a solvent mixture of ethanol and acetonitrile mixture (4800 mL of solvent mixture was prepared by using 2736 mL of absolute ethanol and 2064 mL of acetonitrile) at a temperature in a range of 20-25°C followed by addition of DL-α-tocopherol solution. The mixture was heated to a temperature of 55 to 60°C followed by stirring for 30 to 40 minutes. The hot slurry was filtered, and the resulting solid was washed with 1200 ml (600mLx2) of said solvent mixture. The filtrate was treated with charcoal at a temperature in a range of 40 to 45°C followed by stirring for 55-65 minutes.

The obtained solution was cooled to -12 to -8°C under stirring for 90 minutes, at 100 RPM, resulting in precipitation of further impurities. The resulting mixture was filtered through a jacketed funnel, while maintaining the jacket temperature in a range of -12 to - 8°C followed by addition of DL-α-Tocopherol (prepared by dissolving 0.048 g DL- α-Tocopherol in 30 mL of the solvent mixture prepared earlier) to the filtered solution obtained. The obtained solution was filtered again through a 5 µm and a 0.22 µm polyvinylidene fluoride filter, and then concentrated under vacuum below 35°C until the remaining residue appeared viscous and the remaining viscous mass was dried to obtain 161.8 g (0.27 w/w) of purified egg-yolk lecithin.

### Example 2: Comparison of variable amounts of DMC in the preparation process of purified egg-yolk lecithin

The process of example 1 wherein dimethyl carbonate is used as purification solvent was repeated by using varying quantities of dimethyl carbonate and different process parameters such as temperature and time. The quantity in column 2 is described as a factor, wherein, for example, 3+1 means that 3 times the amount of SFE-EYP in gram was used (in mL) for stirring, and additionally one times the amount was used for washing the filtered amount of SFE-EYP. For example, in row 1, if 150 g of SFE-EYP were treated, they were stirred in 450 ml of DMC and washed with 150 ml. The resulting yield and UV absorbance values are provided below in **Table 4:**

**Table 4:**

| Heating Temp. (°C) | Quantity of DMC (stirring + washing) (based on amount of SFE-EYP) | Stirring time (in hours) | Yield (w/w) | UV Absorbance at 447 nm |
|---|---|---|---|---|
| 40-45 | 3+1 | 1 | 0.27 | 0.18 |
| 40-45 | 6+2 | 1 | 0.27 | 0.13 |
| 40-45 | 6+2 | 2 | 0.27 | 0.15 |
| 55-60 | 6+2 | 1 | 0.26 | 0.13 |
| 55-60 | 8+2 | 1 | 0.26 | 0.14 |

| | | | | |
|---|---|---|---|---|
| It can be seen that the process is robust under a variety of different scenarios, but it seems that the purification is optimized without reduction in yield if an amount of DMC is used that equals six times the amount of SFE-EYP in the initial treatment followed by two times the amount of SFE-EYP in further washing steps. | | | | |

### Example 3: Comparative experiment for the preparation of egg-yolk lecithin with and without DMC

### Example 3.1: Preparation of egg-yolk lecithin using charcoal treatment in step e) without use of DMC (no step b)

240.0 mL of solvent mixture was prepared by using absolute ethanol (136.8 mL) & acetonitrile (103.2 mL) at a temperature in a range of 20-25°C and used for the process. To 170 mL of solvent mixture prepared above, a solution of DL-α-Tocopherol (prepared by dissolving 2.4 mg DL-α-Tocopherol in 5 mL solvent mixture), SFE-EYP (30g) was added under stirring. The mixture was heated to 55 to 60°C followed by stirring for 30-40 minutes. The hot mixture was filtered, and the resulting solid was washed twice with 60 mL (30 mL× 2) of solvent mixture. The combined filtrate was treated with charcoal (1.2 g) at a temperature in a range of 40-45°C followed by stirring 55-65 minutes.

The obtained solution was cooled to -12 to -8°C under stirring for 80-100 minutes. The resulting mixture was filtered at a temperature in a range of -12 to -8°C followed by addition of a solution of DL-α-Tocopherol (prepared by dissolving 2.4 mg DL-α-Tocopherol in 5 mL solvent mixture) to the filtered solution. The obtained solution was concentrated, and the remaining viscous mass was dried under vacuum to obtain 7.9 g (0.26 w/w) of egg-yolk lecithin.

### Example 3.2: Preparation of egg-yolk lecithin without use of DMC (no step b) and without charcoal treatment (no step e)

240.0 mL of solvent mixture was prepared by using absolute ethanol (136.8 mL) & acetonitrile (103.2 mL) at a temperature in a range of 20-25°C and used for the process. To 170 mL of solvent mixture prepared above, a solution of DL-α-Tocopherol (prepared by dissolving 2.4 mg DL-α-Tocopherol in 5 mL solvent mixture), SFE-EYP (30g) was added under stirring. The mixture was heated to 55 to 60°C followed by stirring for 30-40 minutes. The hot mixture was filtered, and the resulting solid was washed twice with 60 mL (30 mL× 2) of solvent mixture.

The obtained solution was cooled to -12 to -8°C under stirring for 80-100 minutes. The resulting mixture was filtered at a temperature in a range of -12 to -8°C. The obtained solution was concentrated, and the remaining viscous mass was dried under vacuum to obtain 8.3 g (0.28 w/w) of egg-yolk lecithin.

### Example 3.3: Preparation of egg-yolk lecithin using DMC in step b) and without charcoal treatment (no step e)

A solution of DL-α-Tocopherol (2.4 mg) in dimethyl carbonate (10 ml), SFE-EYP (30g) and dimethyl carbonate (170 ml) was stirring. The mixture was heated to 40 to 45°C followed by stirring for 55 to 65 minutes. The mixture was filtered, and resulting solid was washed with 60 ml (30 mLx2) of dimethyl carbonate. The solid was dried under vacuum for 4-6 hours.

240.0 mL of solvent mixture was prepared by using absolute ethanol (136.8 mL) & acetonitrile (103.2 mL) at a temperature in a range of 20-25°C and used for the process.

170 mL of solvent mixture, a solution of DL-α-Tocopherol solution (prepared by dissolving 2.4 mg DL-α-Tocopherol in 10 mL solvent mixture) and resulting solid was heated to a temperature of 55 to 60°C followed by stirring for 30-40 minutes. The hot slurry was filtered, and the resulting solid was washed with 60 ml (30mLx2) of said solvent mixture.

The obtained solution was cooled to -12 to -8°C under stirring for 80-100 minutes. The resulting mixture was filtered at a temperature in a range of -12 to -8°C. The obtained solution was concentrated, and the remaining viscous mass was dried under vacuum to obtain 8.2 g (0.27 w/w) of purified egg-yolk lecithin.

### Example 3.4: Preparation of egg-yolk lecithin using DMC in step b) and charcoal treatment in step e)

A solution of DL-α-Tocopherol (2.4 mg) in dimethyl carbonate (10 ml), SFE-EYP (30g) and dimethyl carbonate (170 ml) was stirred. The mixture was heated to 40 to 45°C followed by stirring for 55 to 65 minutes. The mixture was filtered, and resulting solid was washed with 60 ml (30 mLx2) of dimethyl carbonate. The solid was dried under vacuum for 4 to 6 hours.

240.0 mL of solvent mixture was prepared by using absolute ethanol (136.8 mL) and acetonitrile (103.2 mL) at a temperature in a range of 20-25°C and used for the process.

170 mL of solvent mixture, a solution of DL-α-Tocopherol solution (prepared by dissolving 2.4 mg DL-α-Tocopherol in 5 mL solvent mixture) and resulting solid was heated to a temperature of 55 to 60°C followed by stirring for 30 to 40 minutes. The hot slurry was filtered, and the resulting solid was washed with 60 ml (30mLx2) of said solvent mixture. The combined filtrate was stirred with activated charcoal at a temperature in a range of 40 to 45°C for 55-65 minutes.

The obtained solution was cooled to -12 to -8 °C under stirring. The resulting mixture was filtered at a temperature in a range of -12 to -8°C followed by addition of a solution of DL-α-Tocopherol (prepared by dissolving 2.4 mg DL-α-Tocopherol in 5 mL solvent mixture) to the filtered solution obtained above. The obtained solution was concentrated, and the remaining viscous mass was dried under vacuum to obtain 8.0 g (0.26 w/w) of purified egg-yolk lecithin.

The impact of using these different processes for the preparation of egg-yolk lecithin on the purity of the final egg-yolk lecithin (i.e. purified egg-yolk lecithin after all steps of the process described above have been performed), was analysed via High-Performance Liquid Chromatography coupled with a Charged Aerosol Detector, also known as. HPLA-CAD. The assay details are described in Example 6.

The result is shown in Table 5. The table presents the amount of phosphatidylcholine (PC), phosphatidylethanolamine (PE), sphingomyelin (SPH), lysophosphatidyhcholine (LPC) in %w/w. It also presents the yield (in w/w) achieved, and the total amount of colour causing impurities in term of UV-visible spectrophotometer absorption at 447 nm. The sample preparation for the HPLC-UV analysis was performed as follows: 40 mg of sample of purified egg-yolk lecithin were provided in a 20 mL volumetric flask, about 15 mL of ethanol were added and vortexed to dissolve the material completely. The obtained solution was allowed to cool to a temperature in a range of 20-25°C. Then the solution was diluted to volume with ethanol and mixed well to get a solution having 2 mg/mL (0.2 % w/v ) concentration of purified egg yolk-lecithin sample.

**Table 5:**

| Examples | | Yield (w/w) | UV-Vis (447 nm) | HPLC (by CAD) (%w/w) | | | |
|---|---|---|---|---|---|---|---|
| | | | | PC | PE | SPH | LPC |
| Example 3.1 | Charcoal | 0.26 | 0.016 | 77.68 | 6.28 | 1.42 | 2.07 |
| Example 3.2 | - | 0.28 | 0.0633 | 78.56 | 6.45 | 1.39 | 2.03 |
| Example 3.3 | DMC | 0.27 | 0.0118 | 76.37 | 7.60 | 1.42 | 2.01 |
| Example 3.4 | DMC + Charcoal | 0.26 | 0.0036 | 76.53 | 7.17 | 1.45 | 2.00 |

| | | | | | | | |
|---|---|---|---|---|---|---|---|
| Dimethyl carbonate treatment, alone or with charcoalization, enhances the purity of egg-yolk lecithin by lowering UV absorbance at 447 nm significantly. The combination of DMC with charcoal further improves this result, however on cost of yield, reducing yield by 1%. | | | | | | | |

### Example 4: Comparative example for preparation of egg-yolk lecithin using two different solvents (DMC, Acetone)

The process described in example 3.3 is replicated by using acetone instead of dimethyl carbonate.

Egg-yolk lecithin obtained from a process as disclosed in **Example 3.2** (without any solvent), **Example 3.3** (with DMC) and the same process **with acetone** as solvent in step b) of the process, was analysed for yield (ratio of purified egg-yolk lecithin per starting material w/w), PC, PE (in %w/w) by HPLC-CAD and UV absorption at 447 nm. Data are presented in table 6.

Sample preparation for the HPLC-UV analysis was performed as follows: 200 mg of sample of purified egg-yolk lecithin were provided in a 10 mL volumetric flask, about 5 mL of ethanol were added and vortexed to dissolve the material completely. The obtained solution was allowed to cool to a temperature in a range of 20-25°C. Then the solution was diluted to volume with ethanol and mixed well to get a solution having 20 mg/mL (2 % w/v) concentration of purified egg yolk-lecithin sample.

**Table 6:**

| **Solvent used in step b** | Yield of egg-yolk lecithin (w/w) | UV-Vis (447 nm) | PC (%w/w) | PE (%w/w) |
|---|---|---|---|---|
| None | 0.26 | 0.33 | 78.30 | 6.16 |
| Acetone | 0.27 | 0.15 | 78.30 | 7.18 |
| **DMC** | **0.27** | **0.13** | **80.25** | **6.79** |

| | | | | |
|---|---|---|---|---|
| **Results:** An enrichment of PC content and a reduction of PE content was observed in egg-yolk lecithin obtained in dimethyl carbonate experiment with respect to the one obtained by using acetone. Further, the amount of compounds detectable at a UV of 447 nm was clearly reduced when the SFE-EYP was washed before the extraction step, compared to when it was not washed with any solvent. The reduction is even stronger when dimethyl carbonate is used as solvent. | | | | |

### Example 5: HPLC-UV Analysis of purified egg-yolk lecithin

Samples of purified egg-yolk lecithin obtained from different process variations, as well as a commercially obtained sample were analysed via HPLC-UV analysis (High pressure liquid chromatograph (HPLC) with a UV-Visible diode array detector of 200 to 600nm wavelength range, 1 cm cell) at an absorption wavelength of 447 nm to get a better understanding of the reduction of impurities eluting from the column. The type of column used is a Core-shell-(SPP-)-Fused-Core-C18-HPLC-column, known as Ascentis^{®} Express 90 Ǻ C18 (2.7 µm) HPLC column, commercially available, for example from Sigma Aldrich. 250 mg of sample was diluted in 3 mL ethanol by vigorously shaking, before being injected in the column.

The Mobile Phases used were **Mobile phase A** containing a mixture of 700 mL of acetonitrile and 300 mL of water in a 1L bottle which was degassed. **Mobile phase B** contained a mixture of 500 mL of MTBE and 500 mL of methanol in a 1L bottle which was degassed. Ethanol was used as diluent. The blanks were ethanol as well. The following Chromatographic Conditions have been used:

| | | | |
|---|---|---|---|
| Column | Ascentis^{®} Express C18 (150 × 4.6) mm, 2.7 µm | | |
| Flow Rate | 0.4 mL/min. | | |
| Gradient Program | Time (min.) | Mobile Phase A | Mobile Phase B |
| | Initial | 80.0 | 20.0 |
| | 10.0 | 53.0 | 47.0 |
| | 20.0 | 43.0 | 57.0 |
| | 40.0 | 0.0 | 100.0 |
| | 53.0 | 0.0 | 100.0 |
| | 54.0 | 80.0 | 20.0 |
| | 65.0 | 80.0 | 20.0 |
| Column Oven Temperature | 23° C | | |
| Sample Temperature | 25° C | | |
| Detector Wavelength | 447nm | | |
| Injection Volume | 10 µL | | |
| Run Time | 65 min | | |
| Needle wash | Diluent | | |

The area under the curve (AUC) values of the resulting UV chromatogram are provided for impurities eluting at a retention time about 16.2, which is the retention time for zeaxanthin and lutein in this setting, as well as for impurities A, B and C eluting at retention times 18.66, 19.0 and 19.5 in **Table 2** here presented again:

| **Product** | **Lutein & Zeaxanthin RT 16.2** | **Impurity A RT 18.66** | **Impurity B RT 19.0** | **Impurity C RT 19.5** |
|---|---|---|---|---|
| Commercially available Egg- yolk lecithin | 23175 | 727 | 747 | 1197 |
| Egg-yolk lecithin (wherein SFE-EYP is purified using DMC only) | 827 | 0 | 0 | 0 |
| Egg-yolk lecithin (wherein process involves only Charcoal treatment) | 56399 | 3672 | 6464 | 527 |

### Example 6: HPLC-CAD assay for determination of components of purified egg-yolk lecithin

PC, PE, SPH, LPC, PI, LPE & cholesterol content as shown in table 5 was determined by High Performance Liquid Chromatography (HPLC) equipped with CAD. CAD was used to detect non-volatile analytes that do not possess a UV absorbance, by detecting charged aerosol particles that were created when the mobile phase and analyte were evaporated under the following conditions.

The Mobile phases used were the following:
**Mobile phase A** containing acetonitrile.
**Mobile phase B** was prepared by accurately weighing and transferring 1.3 g of ammonium formate into 1 L bottle containing 1000 mL of milli-q water and the mixture was sonicated to mix well. Formic acid was added to adjust the pH to 3.30. The solution was filtered through 0.22 filter paper.
**Mobile phase C** contained a mixture prepared by accurately transferring 500mL of methanol and 500mL of milli-q water into 1 L bottle which was sonicated to mix well and was degassed.

Chloroform: Methanol in a ratio of 2:1 was used as diluent and blank as well.

The following chromatographic conditions have been used:

**Table 7:**

| **For Left pump** | | | | |
|---|---|---|---|---|
| Column | YMC-Pack SIL (250x4.6) mm, 5 µm | | | |
| Flow Rate | 1.5 mL/min. | | | |

| | Flow rate | Time (Min.) | MP-A (%) | MP-B (%) |
|---|---|---|---|---|
| | 1.5 | 0 | 94 | 6 |
| | 1.5 | 1 | 94 | 6 |
| | 1.5 | 20 | 65 | 35 |
| | 1.5 | 20.1 | 94 | 6 |
| | 1.5 | 25 | 94 | 6 |
| | 0.2 | 25.1 | 94 | 6 |
| | 0.2 | 30 | 94 | 6 |
| | 1.5 | 30.1 | 94 | 6 |
| | 1.5 | 40 | 94 | 6 |
| Column Oven Temperature | 23° C | | | |
| Auto Sampler Temperature | 5° C | | | |
| Needle wash | Diluent | | | |
| Detector | CAD | | | |
| Injection Volume | 10 µL | | | |
| Run Time | 40 minutes | | | |
| Rinsing solution | 90:10 :: Methanol : Water | | | |

| **CAD Parameters** | | | | |
|---|---|---|---|---|
| Power function | 1.6 | | | |
| Evaporation temperature | 50°C | | | |
| Signal | 5 Hz | | | |

**Table 8:**

| **For right pump** | | | | | |
|---|---|---|---|---|---|
| Column | YMC-Pack SIL (250x4.6) mm, 5 µm | | | | |
| Flow Rate | 1.5 mL/min. | | | | |

| | Flow rate | Time (Min.) | MP-A (%) | MP-B (%) | MP-C (%) |
|---|---|---|---|---|---|
| | 0.0 | 0.0 | 0 | 0 | 100 |
| | 0.0 | 1 | 0 | 0 | 100 |
| | 0.0 | 20 | 0 | 0 | 100 |
| | 0.0 | 20.1 | 0 | 0 | 100 |
| | 0.0 | 25 | 0 | 0 | 100 |
| | 1.5 | 25.1 | 0 | 0 | 100 |
| | 1.5 | 30 | 0 | 0 | 100 |
| | 0.0 | 30.1 | 0 | 0 | 100 |
| | 0.0 | 40 | 0 | 0 | 100 |
| Column Oven Temperature | 23° C | | | | |
| Auto Sampler Temperature | 5° C | | | | |
| Needle wash | Diluent | | | | |
| Detector | CAD | | | | |
| Injection Volume | 10 µL | | | | |
| Run Time | 40 minutes | | | | |
| Rinsing solution | 90:10 :: Methanol : water | | | | |

| **CAD Parameters** | | | | | |
|---|---|---|---|---|---|
| Power function | | 1.6 | | | |
| Evaporation temperature | | 50 °C | | | |
| Signal | | 5 Hz | | | |

## Claims

1. A process for the preparation of a purified egg-yolk lecithin comprising the steps of:
a. providing supercritical fluid extracted egg-yolk powder as starting material,
b. treating the supercritical fluid extracted egg-yolk powder with a solvent,
c. separating the product obtained in step b) from the solvent used in step b),
d. extracting an egg-yolk lecithin fraction from the product obtained in step c),
e. optionally, treating the egg-yolk lecithin fraction obtained in step d) with charcoal,
f. precipitating impurities from the egg-yolk lecithin fraction obtained in step d) or step e),
g. removing the precipitated impurities from the product obtained in step f),
h. isolating a purified egg-yolk lecithin from the product obtained in step g), and
i. optionally, drying the purified egg-yolk lecithin,
wherein, in step b) the solvent is dimethyl carbonate.

2. The process according to claim 1, wherein any one of the steps selected from the group of step b), step d), step e), and step g) further comprises addition of a radical scavenger.

3. The process according to claim 2, wherein the radical scavenger is DL-α-tocopherol.

4. The process according to any one of the preceding claims wherein treating in step b) comprises adding supercritical fluid extracted egg-yolk powder to a solution of dimethyl carbonate and stirring the obtained mixture at an elevated temperature.

5. The process according to claim 4, wherein the stirring of the obtained mixture is performed at a temperature in the range of 30-60°C, and is maintained for a time interval of 50 to 65 minutes.

6. The process according to any one of the preceding claims, wherein in step b) the amount of dimethyl carbonate is 2 to 10 mL per g of supercritical fluid extracted egg-yolk powder.

7. The process according to any one of the preceding claims, wherein in step d) the egg-yolk lecithin fraction is extracted using a solvent or mixture of several solvents.

8. The process according to any one of the preceding claims, wherein in step e) charcoal treatment is performed at a temperature in the range of 30 to 50°C and at a time interval of 30 minutes to 3 hours.

9. The process according to any one of the preceding claims, wherein in step f) precipitation is achieved by cooling, preferably by cooling at a temperature in a range of-12 to -8°C..

10. A purified egg-yolk lecithin obtained by a process according to any one of claims 1 to 9.

11. The purified egg-yolk lecithin according to claim 10, wherein a UV absorbance value of lutein and zeaxanthin determined as AUC, detected with a UV Vis spectrophotometer at 447 nm, is lower than said UV absorbance of egg-yolk lecithin prepared by a process according to claim 1 but involving no step b).

12. The purified egg-yolk lecithin according to claim 10 or 11, wherein the UV absorbance value of lutein and zeaxanthin is by a factor 10 lower, than the UV absorbance value of egg-yolk lecithin prepared by a process according to claim 1 but involving no step b), but step e) instead.

13. The purified egg-yolk lecithin according to any one of claims 10 to 12, being free from impurities A, B and C, which are eluting at retention times of 18.66 min, 19.0 min and 19.5 min in the HPLC-UV analysis performed at an absorption wavelength of 447 nm, wherein the relative retention time of lutein is 16.2 min.

14. The purified egg-yolk lecithin according to any one of claims 11 to 13, wherein residual amounts of dimethyl carbonate are present, at a level of 50 to 100 ppm, detected by GC-MS.

15. The purified egg-yolk lecithin according to any one of claims 11 to 14, wherein residual amounts of ketone-like organic solvents or chlorinated solvents are below 10 ppm, detected by GC-MS.

16. A lipid emulsion comprising purified egg-yolk lecithin according to any of claims 10 to 15.

17. A method for preparing a lipid emulsion containing propofol, comprising
a) providing an oil phase comprising soybean oil, and propofol;
b) providing an aqueous phase comprising water for injection and a pharmaceutically acceptable tonicity agent, and oleic acid, and/or an agent for pH adjustment;
c) forming a pre-emulsion by mixing the oil phase provided in step a) with the aqueous phase provided in step b);
d) forming an emulsion by high-pressure homogenizing the pre-emulsion obtained in step c); and
e) sterilizing the emulsion obtained in step d), wherein optionally the emulsion is filled into a suitable container before or after sterilization,
wherein purified egg-yolk lecithin according to any one of claims 10 to 15, is added in step a) or step b).

18. The method according to claim 17, wherein the oil phase in step a) further comprises medium chain triglycerides.

19. An oil-in-water emulsion for parenteral administration, comprising:
10 to 20 mg/ml of propofol,
50 to 100 mg/mL soybean oil,
22.5 mg/ml glycerol,
12 mg/ml of purified egg-yolk lecithin according to any of claims 10 to 15, optionally 0.05 mg/ml of disodium edetate anhydrous (equivalent to 0.055 mg of disodium edetate),
sodium hydroxide to adjust pH, and water for injection.

20. The oil-in-water emulsion according to claim 19, wherein the composition further comprises medium-chain triglycerides in a range of 50 to 100 mg/ml.
